# EUROPÄISCHE PATENTSCHRIFT

(11) **EP 4 566 769 B1**
(45) Veröffentlichungstag und Bekanntmachung des Hinweises auf die Patenterteilung: **12.08.2026**
(21) Anmeldenummer: 24212312.3
(22) Anmeldetag: 12.11.2024
(51) Int. Cl.: B25J 13/02, A61B 34/35, A61B 34/00, B25J 18/00, G05G 9/047, G06F 3/01

(54) **HAPTISCHE VORRICHTUNG**
HAPTIC DEVICE
DISPOSITIF HAPTIQUE

(30) Priorität: 28.11.2023 DE 102023133159
(43) Veröffentlichungstag der Anmeldung: 11.06.2025
(73) Patentinhaber: Karl Storz SE & Co. KG, 78532 Tuttlingen (DE)
(72) Erfinder: Klingels, Torben, 78532 Tuttlingen (DE)

(56) Entgegenhaltungen:
- EP-A1- 4 183 313
- EP-B1- 3 463 151
- CN-U- 209 713 135
- DE-T5- 112021 001 860
- JP-A- 2013 049 121

## Beschreibung

Die Erfindung betrifft eine haptische Vorrichtung, die eine Schnittstelle zwischen einem Menschen und einem technischen System bereitstellt.

Aus dem Stand der Technik sind haptische Vorrichtungen mit einem Stellteil als Verbindungs- bzw. Eingabeschnittstelle zwischen Nutzer und einem technischen System bekannt, um z. B. in Roboteranwendungen oder Teleoperationen eine Handbewegung in das System einzugeben. Üblicherweise stellt eine derartige haptische Vorrichtung zumindest drei Bewegungsmöglichkeiten bzw. Freiheitsgrade bereit, die Translations- und/oder Rotationsbewegungen im Raum umfassen.

Solche haptischen Vorrichtungen können als passive Systeme zur reinen Orientierungserfassung ausgebildet sein, wobei vorliegend unter dem Begriff "passiv" verstanden wird, dass Kräfte und/oder Drehmomente, mit denen ein Nutzer das Stellteil bewegt, eine entsprechende Bewegung des technischen Systems hervorrufen können. Alternativ können solche haptischen Vorrichtungen aber auch für Force-Feedback bzw. Kraftrückkopplung als aktive Systeme ausgebildet sein, die eine vom Nutzer über das Stellteil erfahrbare Kraft als Rückmeldung einsetzen. Die dem Benutzer an dem Stellteil angezeigten Kräfte und/oder Drehmomente werden mittels kontrollierter Energiezufuhr von einem oder mehreren Aktoren erzeugt.

Aus WO 2008/003417 A1 ist eine haptische Eingabevorrichtung bekannt, die einen mit einem Handgriff verbundenen Endeffektor aufweist, der über eine parallelkinematische Struktur mit einem Basisteil verbunden ist, wobei die parallelkinematische Struktur translatorische Freiheitsgrade in Bezug auf den Endeffektor bereitstellt. Der Handgriff ist entweder einfach drehbar mit dem Endeffektor verbunden, sodass der Handgriff einen rotatorischen Freiheitsgrad in Bezug auf den Endeffektor aufweist, oder der Handgriff kann mit dem Endeffektor über eine Handgelenkstruktur verbunden sein, die drei rotatorische Freiheitsgrade in Bezug auf den Endeffektor bereitstellt. Dazu weist die Handgelenkstruktur ein Gestänge mit zwei gewinkelten Profilen auf, die schwenkbar miteinander und einerseits mit dem Endeffektor und andererseits mit dem Handgriff verbunden sind, sodass die Schwenkachsen benachbarter Schwenkgelenke orthogonal zueinander sind, und sich die drei Schwenkachsen in einem gemeinsamen Rotationszentrum schneiden, das innerhalb des Handgriffs liegt.

Somit umgibt die Handgelenkstruktur bei Bedienung am Handgriff käfigartig die Hand des Benutzers, was deren Bewegungsbereich einschränken und zu Kollisionen zwischen der Handgelenkstruktur und der Hand des Benutzers oder zwischen den Eingabegeräten und/oder der Umgebung führen kann.

EP 4 183 313 A1 offenbart eine Steuerungsvorrichtung für einen chirurgischen Roboter, die eine kinematische Struktur mit einem Handgriff, einer Basisstruktur und einem Gestänge mit zwei Schwenkarmen aufweist. Die Armsegmente sind so angeordnet, dass sie drei rotatorische Freiheitsgrade bereitstellen, wobei sich die Schwenkachsen in einem gemeinsamen Rotationszentrum schneiden.

Ausgehend von diesem Stand der Technik ist es Aufgabe der vorliegenden Erfindung, eine verbesserte haptische Vorrichtung bereitzustellen, die einen großen Bewegungsbereich zulässt und für die Hand eines Benutzers offen zugänglich ist.

Diese Aufgabe wird durch eine haptische Vorrichtung mit den Merkmalen des Anspruchs 1 gelöst.

Weiterbildungen bzw. bevorzugte Ausführungsformen der haptischen Vorrichtung sind in den Unteransprüchen ausgeführt.

Gemäß der Erfindung weist die haptische Vorrichtung einen Handgriff, eine Basisstruktur und ein Gestänge auf, das den Handgriff mit der Basisstruktur verbindet und drei rotatorische Freiheitsgrade des Handgriffs in Bezug auf die Basisstruktur bereitstellt. Das Gestänge umfasst zwei Schwenkarme, wobei ein erster Schwenkarm durch ein erstes Schwenkgelenk mit der Basisstruktur und durch ein zweites Schwenkgelenk mit dem zweiten Schwenkarm verbunden ist, der durch ein drittes Schwenkgelenk mit dem Handgriff verbunden ist. Eine erste Schwenkachse des ersten Schwenkgelenks, eine zweite Schwenkachse des zweiten Schwenkgelenks und eine dritte Schwenkachse des dritten Schwenkgelenks schneiden sich in einem gemeinsamen Rotationszentrum. Erfindungsgemäß weist der erste Schwenkarm ein erstes Armsegment mit einem Basis-Endabschnitt und einem Kopplungsendabschnitt und ein zweites Armsegment mit einem Kopplungsendabschnitt und einem Arm-Endabschnitt auf. Das erste Schwenkgelenk liegt dabei an dem Basis-Endabschnitt des ersten Armsegments vor, das durch ein erstes Kopplungsgelenk schwenkbar mit dem zweiten Armsegment an den jeweiligen Kopplungsendabschnitten verbunden ist. Das erste Armsegment und das zweite Armsegment sind dabei durch eine erste Getriebevorrichtung derart gekoppelt, dass der Arm-Endabschnitt des zweiten Armsegments auf einer ersten Kreisbahn um eine virtuelle erste Achse bewegt wird, die orthogonal zu der ersten Schwenkachse ist. Analog dazu weist der zweite Schwenkarm ein drittes Armsegment mit einem Basis-Endabschnitt und einem Kopplungsendabschnitt und ein viertes Armsegment mit einem Kopplungsendabschnitt und einem Arm-Endabschnitt auf. Das zweite Schwenkgelenk liegt dann an dem Arm-Endabschnitt des zweiten Armsegments und dem Basis-Endabschnitt des dritten Armsegments vor, und das dritte Schwenkgelenk liegt an dem Arm-Endabschnitt des vierten Armsegments vor. Das dritte Armsegment und das vierte Armsegment sind an ihren Kopplungsendabschnitten durch ein zweites Kopplungsgelenk schwenkbar miteinander verbunden und durch eine zweite Getriebevorrichtung derart gekoppelt, dass der Arm-Endabschnitt des vierten Armsegments auf einer zweiten Kreisbahn um eine virtuelle zweite Achse bewegt wird, die orthogonal zu der zweiten Schwenkachse ist.

Die Erfindung sieht vor, dass die Vorrichtung an dem zweiten Armsegment eine Getriebeerweiterung aufweist, die mit der ersten Getriebevorrichtung und mit einer Plattform gekoppelt ist, die an dem Arm-Endabschnitt angeordnet und mit dem Basis-Endabschnitt des dritten Armsegments gekoppelt ist. Die Getriebeerweiterung ist dazu ausgebildet, die Plattform bei Bewegung mit dem Arm-Endabschnitt entlang der ersten Kreisbahn in Bezug zu einer Ausrichtung der Basisstruktur konstant auszurichten, sodass die erste virtuelle Achse immer orthogonal zu der zweiten virtuellen Achse ist und die Kreisbahn des Arm-Endabschnitts des vierten Armsegments um die zweite virtuelle Achse immer orthogonal zu der Kreisbahn des Arm-Endabschnitts des zweiten Armsegments um die erste virtuelle Achse ist. Auf diese Weise können sich die Arm-Endabschnitte quasi unabhängig voneinander auf den jeweiligen Kreisbahnen bewegen, wobei es unmöglich ist, dass sich die beiden Kreisbahnen in derselben Ebene ausrichten. Um die Zwangsbedingungen für die orthogonalen Kreisbahnen zu erzeugen, kann die Getriebeerweiterung beispielsweise ein dem zweiten Schwenkgelenk zugeordnetes Ausrichtungsbauteil am Arm-Endabschnitt des zweiten Armsegments aufweisen. Dieses Ausrichtungsbauteil, beispielsweise in Form einer Plattform, wird durch die Getriebeerweiterung in einer Weise kontrolliert, dass die Ausrichtung dieser Plattform relativ zur Ausrichtung der Basisstruktur konstant gehalten wird, sodass diese Plattform quasi eine Basisstruktur für den zweiten Schwenkarm darstellt. Dass die Ausrichtung dieser Plattform in Bezug auf die Basisstruktur konstant bleibt, lässt sich beispielsweise verdeutlichen, wenn die Plattform bevorzugt eine Seite oder ein Seitenelement aufweist, die/das zu einer Seite oder einem Seitenelement der Basisstruktur immer, in jeder Position der haptischen Vorrichtung parallel bleibt. Anders ausgedrückt, bezieht sich die konstante Ausrichtung der Plattform in Bezug auf die Ausrichtung der Basisstruktur auf eine Ebene im Raum, die durch die erste Kreisbahn definiert wird, die statisch in Bezug auf die Basisstruktur ist, wobei die Plattform auf dieser Ebene entlang der ersten Kreisbahn bewegt wird.

Die erfindungsgemäße haptische Vorrichtung lässt vorteilhaft einen großen Bewegungsbereich zu, ohne dass eine Hand des Benutzers am Handgriff durch ein Gestänge umschlossen wird. Ein versehentlicher Kontakt der Hand des Benutzers mit dem Gestänge der Vorrichtung beim Ergreifen und Loslassen des Handgriffs findet praktisch kaum statt. Außerdem ist die Kollisionsgefahr mit umgebenden Strukturen oder - bei Verwendung von zwei haptischen Vorrichtungen für die rechte und die linke Hand - zwischen den beiden Vorrichtungen deutlich reduziert. Der Handgriff der erfindungsgemäßen haptischen Vorrichtung kann dabei als Stellteil zur passiven Orientierungserfassung oder zur aktiven Kraftrückkopplung eingesetzt werden.

Nach einer weiteren Ausführungsform der erfindungsgemäßen Vorrichtung ist das Gestänge derart ausgebildet, dass die Kreismittelpunkte der ersten Kreisbahn und der zweiten Kreisbahn der Arm-Endabschnitte des zweiten und vierten Armsegments dem gemeinsamen Rotationszentrum entsprechen, in dem sich folglich die virtuelle erste Achse und die virtuelle zweite Achse schneiden.

Ferner kann jedes Armsegment nach einer weiteren Ausführungsform der erfindungsgemäßen Vorrichtung eine stumpfwinklige V- oder U-Form aufweisen, wobei die jeweiligen Endabschnitte einen stumpfen Winkel einschließen, der beispielsweise dem Innenwinkel eines gleichmäßigen Achtecks entsprechend bei 135° liegen kann. D. h., dass der Basis-Endabschnitt und der Kopplungsendabschnitt des ersten Armsegments, der Kopplungsendabschnitt und der Arm-Endabschnitt des zweiten Armsegments, der Basis-Endabschnitt und der Kopplungsendabschnitt des dritten Armsegments sowie der Kopplungsendabschnitt und der Arm-Endabschnitt des vierten Armsegments jeweils in dem stumpfen Winkel zueinander stehen. Die Armsegmente können einfach gewinkelt sein, d. h. abgeflachte V-Form aufweisen, wobei die beiden Endabschnitte unter dem stumpfen Winkel aufeinander treffen. Alternativ kann jedes Armsegment einen mittleren Abschnitt aufweisen, zu dem die beiden Endabschnitte gleichmäßig abgewinkelt sind, die den stumpfen Winkel einschließen. So können sich eine erste Kopplungsachse des ersten Kopplungsgelenks und eine zweite Kopplungsachse des zweiten Kopplungsgelenks ebenfalls in dem gemeinsamen Rotationszentrum schneiden.

Der Innenwinkel der Armsegmente bzw. der Winkel zwischen den Achsen, die an den Enden eines Armsegments ausgebildet sind (z. B. die erste Schwenkachse und die erste Kopplungsachse am Basis-Endabschnitt und Kopplungsendabschnitt des ersten Armsegments) definiert den maximalen Bewegungsumfang (Range of Motion), wobei kleinere Innenwinkel der Armsegmente bzw. größere Winkel zwischen den Achsen einen größeren Bewegungsumfang bedeuten, aber auch zu einer stärkeren Umschließung der Benutzerhand führen. Bei dem beispielhaften Innenwinkel der Armsegmente von 135° liegt der Winkel zwischen den Achsen eines Armsegments bei **45°,** woraus sich ein maximaler Bewegungsumfang von ± 90° ergibt. Ein größerer Winkel zwischen den Achsen von bspw. 75° (entsprechend einem Innenwinkel von 105°) ergibt einen maximalen Bewegungsumfang von ± 150°.

Weitere Ausführungsformen der erfindungsgemäßen Vorrichtung beziehen sich darauf, dass die erste Getriebevorrichtung und die zweite Getriebevorrichtung unabhängig voneinander aus einer Gruppe ausgewählt sein können, die eine Riemengetriebevorrichtung, eine Kettengetriebevorrichtung und eine Kegelgetriebevorrichtung sowie Kombinationen davon aufweist.

In einer bevorzugten Weiterbildung der erfindungsgemäßen Vorrichtung wird die erste Getriebevorrichtung oder die zweite Getriebevorrichtung oder werden beide Getriebevorrichtungen durch jeweils eine Riemengetriebevorrichtung bereitgestellt. Eine als erste Getriebevorrichtung eingesetzte Riemengetriebevorrichtung kann eine erste Riemenscheibe an dem Basis-Endabschnitt des ersten Armsegments, eine zweite Riemenscheibe an dem Kopplungsendabschnitt des ersten Armsegments und einen Riemen aufweisen, der über die erste Riemenscheibe und die zweite Riemenscheibe läuft. Eine Achse der ersten Riemenscheibe an dem Basis-Endabschnitt des ersten Armsegments entspricht der ersten Schwenkachse, und eine Achse der zweiten Riemenscheibe an dem Kopplungsendabschnitt des ersten Armsegments entspricht der ersten Kopplungsachse. Durch die Kopplungen der ersten Riemenscheibe mit dem ersten Schwenkgelenk und der zweiten Riemenscheibe mit dem ersten Kopplungsgelenk kann der Riemen Drehbewegungen des ersten Armsegments in Bezug zur Basisstruktur auf das zweite Armsegment übertragen. Die Abstimmung des Übersetzungsverhältnisses der Getriebevorrichtung sorgt dann dafür, dass sich der Arm-Endabschnitt des zweiten Schwenkarms immer auf der ersten Kreisbahn bewegt.

Entsprechend kann eine als zweite Getriebevorrichtung eingesetzte Riemengetriebevorrichtung eine erste Riemenscheibe an dem Basis-Endabschnitt des dritten Armsegments, eine zweite Riemenscheibe an dem Kopplungsendabschnitt des dritten Armsegments und einen Riemen aufweisen, der über die erste Riemenscheibe und die zweite Riemenscheibe läuft. Dabei entspricht eine Achse der ersten Riemenscheibe an dem Basis-Endabschnitt des dritten Armsegments der zweiten Schwenkachse, und eine Achse der zweiten Riemenscheibe an dem Kopplungsendabschnitt des dritten Armsegments entspricht der zweiten Kopplungsachse. Hierbei sorgt die Kopplung der ersten Riemenscheibe mit dem zweiten Schwenkgelenk und der zweiten Riemenscheibe mit dem zweiten Kopplungsgelenk bei abgestimmtem Übersetzungsverhältnis dafür, dass der Riemen Drehbewegungen des dritten Armsegments in Bezug zu dem Arm-Endabschnitt des zweiten Armsegments auf das vierte Armsegment so überträgt, dass sich der Arm-Endabschnitt des vierten Schwenkarms immer auf der zweiten Kreisbahn bewegt.

Eine weitere vorteilhafte Ausführungsform der erfindungsgemäßen Vorrichtung sieht dabei vor, dass der Riemen der jeweiligen Riemengetriebevorrichtung ein Profil aufweist und aus einer Gruppe ausgewählt ist, die zumindest einen Zahnriemen und einen Synchronrundriemen umfasst, der ein mit einem Wendelelement verbundener Rundriemen ist. Entsprechend weisen die erste Riemenscheibe und die zweite Riemenscheibe eine Profilierung auf, die mit dem Profil des Riemens korrespondiert und die aus einer Gruppe ausgewählt ist, die zumindest eine Zahnung zum Eingriff mit dem Zahnriemen und eine Rillensegmentierung zum Eingriff dem Synchronrundriemen umfasst, sodass die Riemengetriebevorrichtung eine schlupffreie oder zumindest schlupfarme Kraftübertragung bereitstellt.

Außerdem kann die Riemengetriebevorrichtung nach einer weiteren Ausführungsform der erfindungsgemäßen haptischen Vorrichtung zumindest ein Rollenelement aufweisen, das zur Führung des Riemens zwischen der ersten Riemenscheibe und der zweiten Riemenscheibe ausgebildet und aus einer Gruppe ausgewählt ist, die zumindest eine Umlenkrolle und eine Spannrolle umfasst.

Nach noch einer weiteren Ausführungsform der erfindungsgemäßen Vorrichtung kann die Getriebeerweiterung zur Kopplung mit der ersten Getriebevorrichtung und dem Ausrichtungsbauteil aus einer Gruppe ausgewählt sein, die eine Riemengetriebevorrichtung, eine Kettengetriebevorrichtung, eine Zahnradgetriebevorrichtung, eine kombinierte Riemen-Zahnradgetriebevorrichtung und eine kombinierte Ketten-Zahnradgetriebevorrichtung aufweist.

In einer beispielhaften Ausführungsform der erfindungsgemäßen Vorrichtung wird die Getriebeerweiterung durch die kombinierte Riemen-Zahnradgetriebevorrichtung bereitgestellt, die eine erste Riemenscheibe an einem Zusatzabschnitt des zweiten Armsegments aufweist. Der Zusatzabschnitt befindet sich auf einer von dem Arm-Endabschnitt abgewandten Seite des Kopplungsendabschnitts. Ferner weist diese Getriebeerweiterung eine zweite Riemenscheibe an dem Arm-Endabschnitt des zweiten Armsegments und einen Riemen auf, der über die erste Riemenscheibe und die zweite Riemenscheibe der kombinierte Riemen-Zahnradgetriebevorrichtung läuft. Eine Achse der ersten Riemenscheibe an dem Zusatzabschnitt des zweiten Armsegments ist dabei parallel zu der ersten Kopplungsachse, und eine Achse der zweiten Riemenscheibe an dem Arm-Endabschnitt des zweiten Armsegments entspricht der zweiten Schwenkachse. Dabei steht ein erstes Zahnrad, das koaxial mit der ersten Riemenscheibe der kombinierten Riemen-Zahnradgetriebevorrichtung drehfest verbunden ist, mit einem zweiten Zahnrad in Eingriff, das koaxial mit der zweiten Riemenscheibe der ersten Getriebevorrichtung drehfest verbunden ist.

Dabei kann nach einer weiteren Ausführungsform der erfindungsgemäßen Vorrichtung auch der Riemen der kombinierten Riemen-Zahnradgetriebevorrichtung, die die Getriebeerweiterung bereitstellt, ein Profil aufweisen und aus einer Gruppe ausgewählt sein, die zumindest einen Zahnriemen und einen Synchronrundriemen umfasst, der ein mit einem Wendelelement verbundener Rundriemen ist. Für schlupffreie bzw. schlupfarme Kraftübertragung haben dann auch die die erste Riemenscheibe und die zweite Riemenscheibe der Getriebeerweiterung eine Profilierung, die mit dem Profil des Riemens korrespondiert und aus einer Gruppe ausgewählt ist, die zumindest eine Zahnung zum Eingriff mit dem Zahnriemen und eine Rillensegmentierung zum Eingriff mit dem Synchronrundriemen umfasst.

Ferner kann auch die kombinierte Riemen-Zahnradgetriebevorrichtung in einer Weiterbildung der erfindungsgemäßen Vorrichtung zumindest ein Rollenelement aufweisen, das zur Führung des Riemens zwischen der ersten Riemenscheibe und der zweiten Riemenscheibe der Getriebeerweiterung ausgebildet ist und das aus einer Gruppe ausgewählt ist, die zumindest eine Umlenkrolle und eine Spannrolle umfasst.

Weitere Ausführungsformen der erfindungsgemäßen haptischen Vorrichtung beziehen sich darauf, dass die haptische Vorrichtung als passives System zur reinen Orientierungs- bzw. Richtungserfassung oder als aktives System für Force-Feedback bzw. Kraftrückkopplung ausgebildet sein kann, um einem Benutzer eine Kraftrückmeldung zu geben. Die dem Benutzer angezeigten Kräfte und/oder Drehmomente werden mittels kontrollierter Energiezufuhr von einem oder mehreren Aktoren erzeugt, die beispielsweise Capstan-Antriebe oder Direktantriebe auf Basis von DC- oder EC-Motoren sein können.

Weitere Ausführungsformen der erfindungsgemäßen haptischen Vorrichtung sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- Fig. 1: eine perspektivische Ansicht der haptischen Vorrichtung nach einer erfindungsgemäßen Ausführungsform in Ruheposition,
- Fig. 2: eine perspektivische Ansicht der haptischen Vorrichtung aus Fig. 1 in einer Arbeitsposition,
- Fig. 3: eine teiltransparente Detailansicht der Armsegmente des ersten Schwenkarms der haptischen Vorrichtung in der Arbeitsposition aus Fig. 2, und
- Fig. 4 bis 7: schematische perspektivische Ansichten der haptischen Vorrichtung aus Fig. 1 in verschiedenen Arbeitspositionen.

Die Erfindung bezieht sich auf eine haptische Vorrichtung, die als haptisches Eingabegerät für ein chirurgisches Teleoperationssystem geeignet ist. Sie vermeidet nicht nur eine vollständige Umschließung der Hand des Benutzers, sondern stellt auch einen großen Bewegungsbereich bereit.

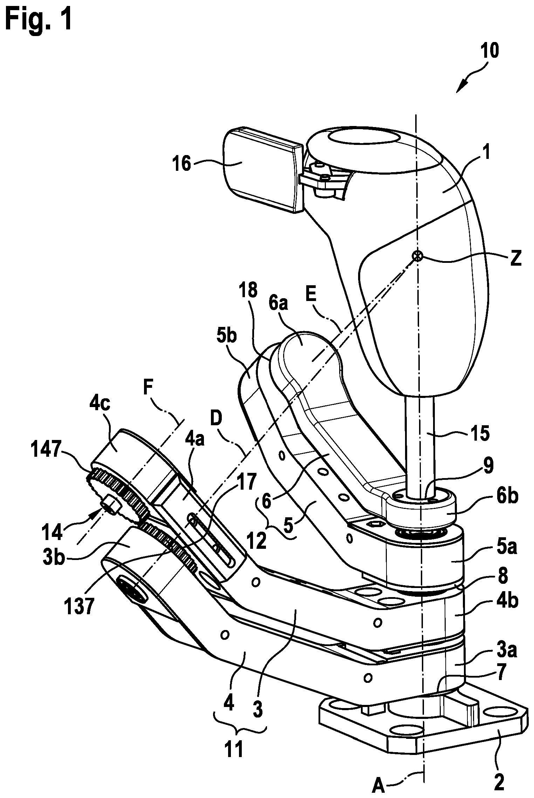

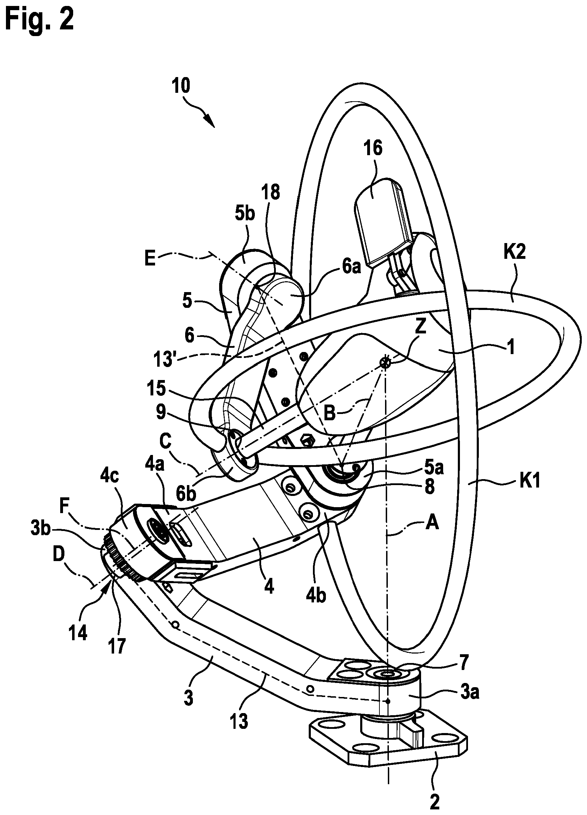

Die Figuren 1 und 2 zeigen eine erfindungsgemäße haptische Vorrichtung 10. Diese weist ein Gestänge auf, das einen Handgriff 1 mit einer Basisstruktur 2 verbindet und drei rotatorische Freiheitsgrade des Handgriffs 1 in Bezug auf die Basisstruktur 2 bereitstellt. Der dargestellte Handgriff 10 weist mit einem Taster 16 ein zusätzliches Betätigungselement als Benutzerschnittstelle auf, die eine von der Orientierung des Handgriffs unabhängige Eingabe mittels Fingerdruck gestattet.

Nach einer nicht figurativ gezeigten Ausführungsform der erfindungsgemäßen haptische Vorrichtung kann der Handgriff einer solchen Vorrichtung ohne ein solches Betätigungselement oder mit mehr als einem solchen Betätigungselement ausgebildet sein, um unterschiedliche Eingaben tätigen zu können.

Die Basisstruktur der erfindungsgemäßen haptischen Vorrichtung kann entweder ortsfest im Raum oder als Endeffektor am Ende einer Positionierungsvorrichtung angeordnet sein. Eine Positioniervorrichtung kann z. B. einer Kinematik zur Bereitstellung translatorischer Freiheitsgrade in Bezug auf die als Endeffektor ausgebildete Basisstruktur dienen.

Das Gestänge besteht aus vier Armsegmenten 3, 4, 5, 6, die unter Ausbildung einer Gelenkarmkette gelenkig miteinander, sowie mit dem Handgriff 1 und der Basisstruktur 2 verbunden sind. Hierbei werden ein erstes Armsegment 3 und ein zweites Armsegment 4 als erster Schwenkarm 11, sowie ein drittes Armsegment 5 und ein viertes Armsegment 6 als zweiter Schwenkarm 12 definiert. Das erste Armsegment 3 hat einenends einen Basis-Endabschnitt 3a, an dem das erste Armsegment 3 mit der Basisstruktur 2 durch ein erstes Schwenkgelenk 7 verbunden ist, das eine erste Schwenkachse A definiert. An seinem anderen Ende hat das erste Armsegment 3 einen Kopplungsendabschnitt 3b zur schwenkbaren Verbindung durch ein erstes Kopplungsgelenk 17 mit dem zweiten Armsegment 4 an dessen Kopplungsendabschnitt 4a. An dessen anderen Ende, dem Arm-Endabschnitt 4b, verbindet ein zweites Schwenkgelenk 8, das eine zweite Schwenkachse B definiert, das zweite Armsegment 4 mit dem dritten Armsegment 5 an dessen Basis-Endabschnitt 5a. Das dritte Armsegment 5 hat an seinem anderen Ende einen Kopplungsendabschnitt 5b zur schwenkbaren Verbindung durch ein zweites Kopplungsgelenk 18 mit dem vierten Armsegment 6 an dessen Kopplungsendabschnitt 6a. Das andere Ende des vierten Armsegments 6 stellt den Arm-Endabschnitt 6b dar, an dem das vierte Armsegment 6 durch ein drittes Schwenkgelenk 9 mittels der Handgriffachse 15 mit dem Handgriff 1 verbunden ist. Das dritte Schwenkgelenk 9 definiert eine dritte Schwenkachse C, die sich in einer Arbeitsposition der haptischen Vorrichtung 10 mit der der ersten Schwenkachse A und der zweiten Schwenkachse B in einem Punkt schneidet, der in dem Handgriff 1 liegt und dessen Rotationszentrum Z darstellt, wie in Figur 2 zu sehen ist, die die haptische Vorrichtung 10 in einer Arbeitsposition zeigt. In der in Figur 1 dargestellten Ruheposition der haptischen Vorrichtung 10 ist die erste Schwenkachse A koaxial mit der zweiten und dritten Schwenkachse B, C, da die Basis-Endabschnitte 3a, 5a sowie die Arm-Endabschnitte 4b, 6b direkt übereinander zu liegen kommen. Dabei sind die Armsegmente 3, 4 des ersten Schwenkarms 11 und die orthogonal dazu ausgerichteten Armsegmente 5, 6 des zweiten Schwenkarms 12 platzsparend übereinander angeordnet.

Die Armsegmente 3, 4; 5, 6 weisen im vorliegenden Beispiel eine stumpfwinkelig aufgeweitete U-Form auf, wobei die beiden Endabschnitte 3a, 3b; 4a, 4b; 5a, 5b; 6a, 6b jedes Armsegments 3, 4; 5, 6 gleichermaßen von einem mittleren (unbezeichneten) Armabschnitt des jeweiligen Armsegments 3, 4; 5, 6 abgewinkelt sind. Die beiden Endabschnitte 3a, 3b; 4a, 4b; 5a, 5b; 6a, 6b jedes Armsegments 3, 4; 5, 6 schließen hierbei einen stumpfen Winkel ein, der im vorliegenden Beispiel in etwa dem Innenwinkel eines gleichmäßigen Achtecks von 135° entspricht. D. h., dass der Basis-Endabschnitt 3a und der Kopplungsendabschnitt 3b des ersten Armsegments 3 in einem Winkel von ca. 135° zueinander stehen, ebenso wie der Kopplungsendabschnitt 4a und der Arm-Endabschnitt 4b des zweiten Armsegments 4. Entsprechendes gilt für den Basis-Endabschnitt 5a und den Kopplungsendabschnitt 5b des dritten Armsegments 5, sowie den Kopplungsendabschnitt 6a und den Arm-Endabschnitt 6b des vierten Armsegments 6.

So stellen das erste Kopplungsgelenk 17 zwischen dem ersten und zweiten Armsegment 3, 4 und das zweite Kopplungsgelenk 18 zwischen dem dritten und vierten Armsegment 5, 6 eine erste Kopplungsachse D und eine zweite Kopplungsachse E bereit, die sich, wie in Figur 1 dargestellt, ebenfalls in einem Punkt schneiden, der dem gemeinsamen Rotationszentrum Z entspricht.

Um die drei rotatorischen Freiheitsgrade des Handgriffs 1 in Bezug auf die Basisstruktur 2 bereitzustellen, sorgen eine erste Getriebevorrichtung 13, die die beiden Armsegmente 3, 4 des ersten Schwenkarms 11 koppelt, und eine zweite Getriebevorrichtung 13', die die beiden Armsegmente 5, 6 des zweiten Schwenkarms 12 koppelt, für mechanische Zwangsbedingungen, die in einer gebundenen Bewegung der jeweiligen Arm-Endabschnitte 4a, 6a resultieren. Durch die erste Getriebevorrichtung 13 bewegt sich der Arm-Endabschnitt 4b am zweiten Armsegment 4 des ersten Schwenkarms 11 auf eine erste Kreisbahn K1 um eine virtuelle Achse, die orthogonal zur ersten Schwenkachse A ist. Und durch die zweite Getriebevorrichtung 13' bewegt sich der Arm-Endabschnitt 6b am vierten Armsegment 6 des zweiten Schwenkarms 12 auf einer zweiten Kreisbahn K2 um eine virtuelle Achse, die orthogonal zu der zweiten Schwenkachse B ist. In Figur 2 sind die Kreisbahnen K1, K2 und die Schwenkachsen A, B, C visualisiert, weshalb auf eine Darstellung der virtuellen Achsen der Übersichtlichkeit wegen verzichtet wurde. Die in der Darstellung vertikale erste Kreisbahn K1 des Arm-Endabschnitts 4b am zweiten Armsegment 4 ist in Bezug auf die Basisstruktur 2 statisch, kann aber mit der Basisstruktur 2 um die erste Schwenkachse A rotiert werden. Die zweite Kreisbahn K2, die in der Darstellung annähernd horizontal verläuft, wird durch eine Getriebeerweiterung 14 immer orthogonal zur ersten Kreisbahn K1 gehalten, dreht sich jedoch innerhalb dieser um das gemeinsame Rotationszentrum Z aller Achsen, abhängig von der aktuellen Position des ersten Arm-Endabschnitts 4b auf der ersten Kreisbahn K1. Das gemeinsame Rotationszentrum Z entspricht folglich den Kreismittelpunkten der beiden Kreisbahnen K1, K2.

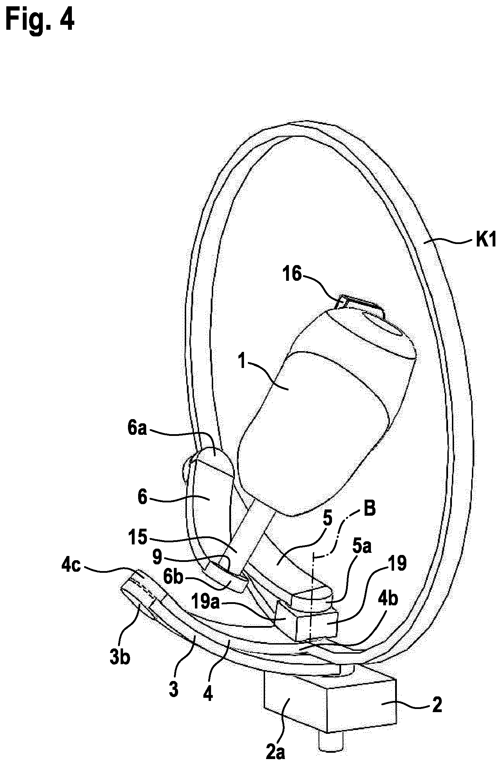

Die Getriebeerweiterung 14 an dem zweiten Armsegment 4 kontrolliert eine in Fig. 2 nicht dargestellte Plattform 19 an dem Arm-Endabschnitt 4b, die mit dem Basis-Endabschnitt 5a gekoppelt ist. Dabei sorgt die Getriebeerweiterung 14 dafür, dass die Ausrichtung der mit dem Arm-Endabschnitt 4b auf der ersten Kreisbahn K1 bewegten Plattform 19 relativ zur Ausrichtung der Basisstruktur 2 konstant bleibt, wie nachfolgend im Zusammenhang mit Fig. 4 bis 7 erläutert wird. Auf diese Weise ist die erste virtuelle Achse immer orthogonal zu der zweiten virtuellen Achse, bzw. die zweite Kreisbahn K2 immer orthogonal zu der ersten Kreisbahn K1. Da die zweite Schwenkachse B, um die sich der zweite Schwenkarm 12 bewegt, durch die ausgerichtete Plattform 19 immer in der Ebene der ersten Kreisbahn K1 liegt, können die Kreisbahnen K1, K2 niemals identisch sein, wodurch der Verlust eines Freiheitsgrades verhindert wird. Die relative Ausrichtung der Basis-Endabschnitte 5a, 3a zueinander ist auch in der Ruheposition in Figur 1 zu sehen. Eine entsprechende Getriebeerweiterung könnte auch an dem vierten Armsegment 6 verwendet werden, ist aber nicht erforderlich, da der Handgriff 1 einfach direkt über die Handgriffachse 15 um die dritte Schwenkachse C drehbar gelagert ist, die immer in der Ebene der zweiten Kreisbahn K2 liegt.

Die mechanischen Verbindungen der ersten und zweiten Getriebevorrichtungen 13, 13' und der Getriebeerweiterung 14 können grundsätzlich verschiedene Getriebeelemente aufweisen. Diese schließen Zahnriemen, Ketten, Synchronrundriemen oder Kegelräder ein, sind aber nicht beschränkt darauf.

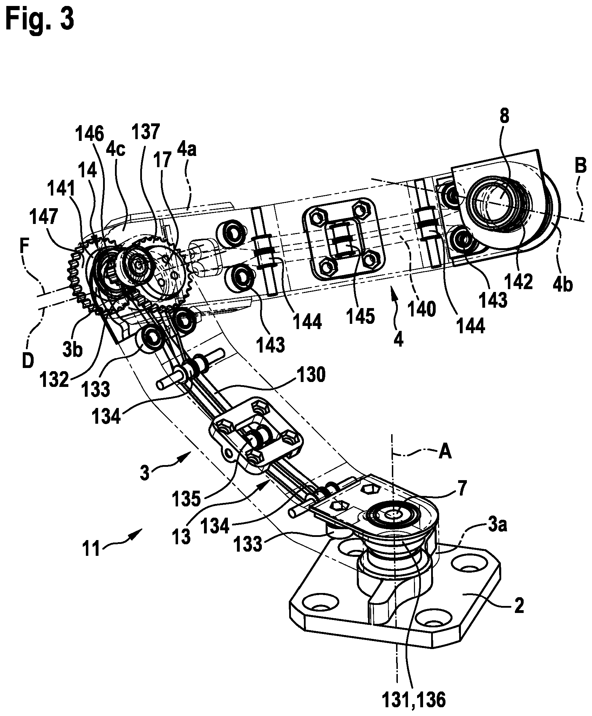

Bei der in den Figuren gezeigten Ausführungsform sind die Getriebevorrichtungen 13, 13' als Riemengetriebevorrichtungen 13, 13' und die Getriebeerweiterung 14 als kombinierte Riemen-Zahnradgetriebevorrichtung 14 ausgeführt. Die erste Riemengetriebevorrichtung 13 und die Getriebeerweiterung 14 sind in Figur 3 detailliert dargestellt, die nicht im Detail dargestellte zweite Riemengetriebevorrichtung 13' ist analog zur ersten Riemengetriebevorrichtung 13 aufgebaut.

Wie in Figur 3 zu sehen ist, sind die Armsegmente 3, 4 als Gehäuse ausgebildet, in oder an denen die Getriebeelemente der ersten Riemengetriebevorrichtung 13 und der Getriebeerweiterung 14 angeordnet bzw. gelagert sind. Die erste Riemengetriebevorrichtung 13 erstreckt sich durch das erste Armsegment 3 und die Getriebeerweiterung 14 durch das zweite Armsegment 4 des ersten Schwenkarms 11. Zur Anordnung bzw. Lagerung der Getriebeelemente der zweiten Getriebevorrichtung 13' ist auch das dritte Armsegment 5 als Gehäuse ausgebildet. Das vierte Armsegment 6 hingegen benötigt lediglich eine Lagerungsstruktur an dem Kopplungsendabschnitt 6a zur Ausbildung der schwenkbaren Verbindung mit dem dritten Armsegment 5 und eine Lagerungsstruktur an dem Arm-Endabschnitt 6b zur Ausbildung des dritten Schwenkgelenks 9 mit der Handgriffachse 15, da in dem zweiten Schwenkarm 12 vorzugsweise auf eine Getriebeerweiterung 14 verzichtet werden kann.

Die erste Riemengetriebevorrichtung 13 weist eine erste Riemenscheibe 131 auf, die innerhalb des Gehäuses des ersten Armsegments 3 an dem Basis-Endabschnitt 3a angeordnet und über das erste Schwenkgelenk 7 mit der Basisstruktur 2 gekoppelt ist, wobei eine Achse der ersten Riemenscheibe 131 der ersten Schwenkachse A entspricht. Eine zweite Riemenscheibe 132 befindet sich an dem Kopplungsendabschnitt 3b des ersten Armsegments 3 und ist über das erste Kopplungsgelenk 17 mit dem zweiten Armsegment 4 gekoppelt, wobei eine Achse der zweiten Riemenscheibe 132 der ersten Kopplungsachse D entspricht. Ein Riemen 130, der durch das erste Armsegment über die erste Riemenscheibe 131 und die zweite Riemenscheibe 132 läuft, überträgt Drehbewegungen des ersten Schwenkarms 11 in Bezug zur Basisstruktur 2 auf das zweite Armsegment 4. Die Auswahl des Übersetzungsverhältnisses sorgt für die Zwangsbedingungen, damit sich der Arm-Endabschnitt 4b des ersten Schwenkarms 11 auf der ersten Kreisbahn K1 bewegt.

Eine Riemengetriebevorrichtung als zweite Getriebevorrichtung 13' weist analog dazu eine erste Riemenscheibe an dem Basis-Endabschnitt 5a des dritten Armsegments 5, eine zweite Riemenscheibe an dem Kopplungsendabschnitt 5b des dritten Armsegments 5 und einen Riemen auf, der über die beiden Riemenscheiben läuft. Dabei entspricht eine Achse der ersten Riemenscheibe, die über das zweite Schwenkgelenk 8 mit dem zweiten Armsegment 4 gekoppelt ist, der zweiten Schwenkachse B, und eine Achse der zweiten Riemenscheibe, die über das zweite Kopplungsgelenk 18 mit dem vierten Armsegment 6 gekoppelt ist, entspricht zweiten Kopplungsachse E. Damit werden Drehbewegungen des zweiten Schwenkarms 12 in Bezug auf das Arm-Ende 4b des zweiten Armsegments 4 auf das vierte Armsegment 6 übertragen, sodass sich bei passender Auswahl des Übersetzungsverhältnisses der Arm-Endabschnitt 6b des zweiten Schwenkarms 11 auf der zweiten Kreisbahn K2 bewegt.

Damit der zweite Schwenkarm 12 unabhängig von dem ersten Schwenkarm 11 bewegt werden kann, ist die konstante Ausrichtung der mit dem Basis-Endabschnitt 5a gekoppelten Plattform 19 relativ zur Ausrichtung der Basisstruktur 2 erforderlich. Um den Basis-Endabschnitt 5a an dem Arm-Ende 4b des zweiten Armsegments 4 passiv auszurichten, weist das zweite Armsegment 4 die Getriebeerweiterung 14 auf. Dazu weist das zweite Armsegment 4 einen Zusatzabschnitt 4c auf einer von dem Arm-Endabschnitt 4b abgewandten Seite des Kopplungsendabschnitts 4a auf.

Die haptische Vorrichtung 10 beschreibt in verschiedenen Arbeitspositionen mit dem Basis-Endabschnitt 5a des dritten Armsegments 5 bzw. dem zweiten Schwenkgelenk 8 die virtuelle Kreisbahn K1, wie in Figuren 4 bis 7 zu sehen ist. In dem zweiten Schwenkgelenk 8 ist am Arm-Endabschnitt 4b des zweiten Armsegments 4 ein in Bezug auf die zweite Schwenkachse B drehbar gelagertes Ausrichtungsbauteil in Form einer Plattform 19 angeordnet, die mit der in Fig. 4 bis 7 nicht figurativ dargestellten Getriebeerweiterung 14 am Zusatzabschnitt 4c (in den Figuren 1 bis 3) operativ gekoppelt ist und durch diese kontrolliert wird.

In nicht figurativ dargestellten Varianten kann die Plattform auch ein gerundetes Bauteil sein. Stets gleich bleibt, dass die mit dem Arm-Endabschnitt 4b entlang der ersten Kreisbahn K1 bewegte Plattform 19 ein in Bezug auf die Basisstruktur 2 konstant ausgerichtetes Bauteil ist. In Figuren 4 bis 7 ist dies dargestellt durch die Plattform 19, deren parallel zu der ersten Kreisbahn K1 bewegte Seite 19a zu einer Seite 2a der Basisstruktur 2 immer parallel orientiert ist, und zwar in jeder möglichen Position der haptischen Vorrichtung 10. Dies erzeugt die Zwangsbedingung, dass die zweite Kreisbahn K2 in Bezug zu der ersten Kreisbahn K1 stets im rechten Winkel steht.

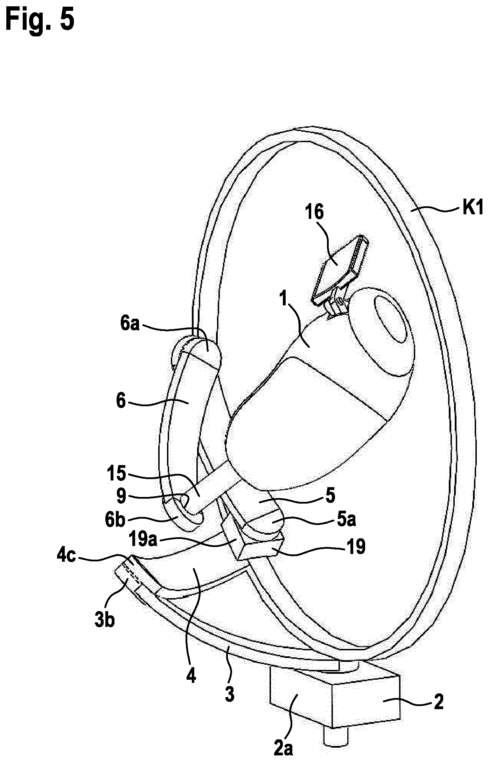

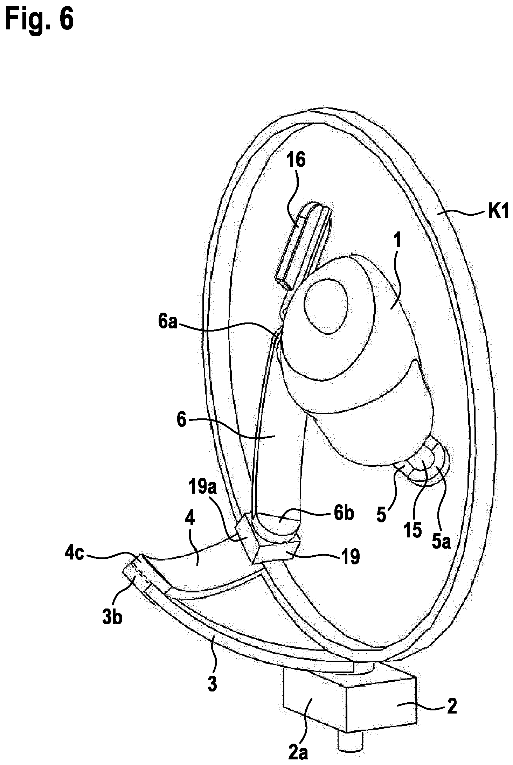

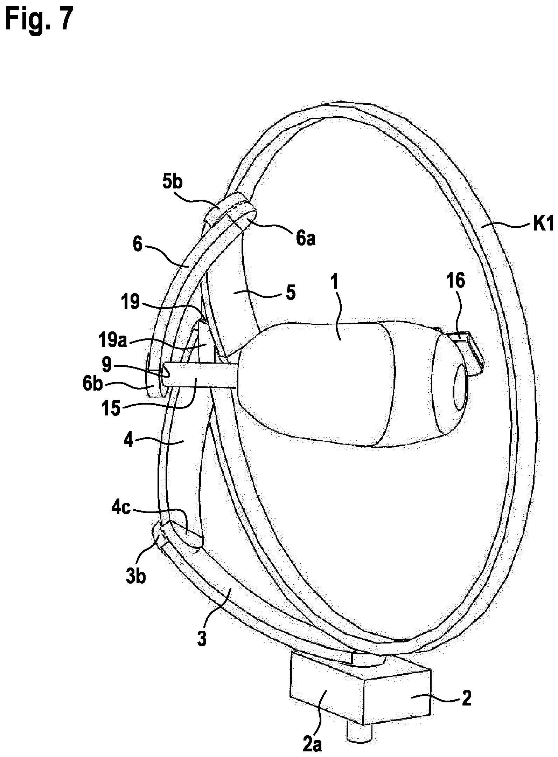

Ausgehend von der Ruheposition nach Figur 1 verschwenkt in Figur 4 das dritte Armsegment 5 und das vierte Armsegment 6. Wechselnd zu Figur 5 verschwenkt das zweite Armsegment 4, so dass die Plattform 19 entlang der ersten Kreisbahn K1 verfährt. Dann verschwenkt das vierte Armsegment 6 in Figur 6 nach hinten, die Plattform 19 verbleibt an ihrer Position aus Figur 5. Schließlich wird der Handgriff 1 in Figur 7 zur linken Figurenseite gedreht und die Armsegmente 3, 4 und 5 verschenken auseinander, so dass die Plattform 19 in der ersten Kreisbahn K1 nach weiter oben verfährt. Die Seite 19a der Plattform 19 liegt immer parallel zu der Seite 2a der Basisstruktur 2.

Die in Fig. 3 beispielhaft als kombinierte Riemen-Zahnradgetriebevorrichtung 14 ausgebildete Getriebeerweiterung 14 weist eine erste Riemenscheibe 141 auf, die an dem Zusatzabschnitt 4c in dem Gehäuse des zweiten Armsegments 4 angeordnet ist. Die Achse F der ersten Riemenscheibe 141 ist dabei parallel zu der ersten Kopplungsachse D des ersten Kopplungsgelenks 17 zwischen dem ersten Armsegment 3 und dem zweiten Armsegment 4. Die erste Riemenscheibe 141 der Getriebeerweiterung 14 ist mit der zweite Riemenscheibe 132 der ersten Getriebevorrichtung 13 gekoppelt. Dazu ist die erste Riemenscheibe 141 koaxial mit einem erstes Zahnrad 147 drehfest verbunden, das an dem Zusatzabschnitt 4c auf einer Gehäuseseite des zweiten Armsegments 4 angeordnet ist, die dem ersten Armsegment 3 zugewandt ist. Dieses erste Zahnrad 147 steht mit einem zweiten Zahnrad 137 in Eingriff, das koaxial mit der zweiten Riemenscheibe 132 der ersten Getriebevorrichtung 13 drehfest verbunden ist und an dem Kopplungsabschnitt 3b auf einer Gehäuseseite des ersten Armsegments 3 angeordnet ist, die dem zweiten Armsegment 4 zugewandt ist. Die erste Riemenscheibe 141 der Getriebeerweiterung 14 steht über einen Riemen 140 mit einer zweiten Riemenscheibe 142 in Verbindung, die an dem Arm-Endabschnitt 4b des zweiten Armsegments 4 angeordnet ist und mittels der Plattform 19 (vgl. Fig. 4 bis 7) über das Schwenkgelenk 8 mit dem dritten Armsegment 5 gekoppelt ist, wobei die Achse der zweiten Riemenscheibe 142 der zweiten Schwenkachse B entspricht.

Bei den Riemen 130, 140 der Getriebevorrichtung 13 und der Getriebeerweiterung 14 handelt es sich im dargestellten Beispiel um schlupffreie Zahnriemen, wenngleich in den Figuren auf die Darstellung der Zahnung der Riemen 130, 140 verzichtet wurde. Die Riemenscheiben 131, 132, 141, 142 der Getriebevorrichtung 13 und der Getriebeerweiterung 14 weisen eine entsprechende Zahnung 136, 146 auf, die in Figur 3 an den jeweiligen ersten Riemenscheiben 131, 141 zu sehen ist.

Zur platzsparenden Führung der Riemen 130, 140 durch die abgewinkelten Armsegmente 3, 4 weist sowohl die Riemengetriebevorrichtung 13 im ersten Armsegment 3 als auch die Getriebeerweiterung 14 im zweiten Armsegment 4 mehrere Rollenelemente auf: Jeweils zwei zu den Riemenscheiben 131, 132, 141, 142 achsparallele Umlenkrollen 133 sorgen für eine Zusammenführung des an den Riemenscheiben 131, 132, 141, 142 umlaufenden Riemens 130, 140. Im Übergangsbereich von den abgewinkelten Endabschnitten 3a, 3b; 4a, 4b zu dem mittleren Armabschnitt des jeweiligen Armsegments 3, 4 sind jeweils zwei Umlenkrollen 134, 144 angeordnet, deren Achse orthogonal zu der Achse der jeweiligen Riemenscheibe 131, 132, 141, 142 verläuft, sodass der zusammengeführte Riemen 130, 140 zwischen den parallelen Umlenkrollen 133, 143 und den orthogonalen Umlenkrollen 134, 144 geschränkt wird. In dem mittleren Armabschnitt des jeweiligen Armsegments 3, 4 sorgen Spannrollen 135, 145 an den geschränkten Riemen 130, 140 für die gewünschte Vorspannkraft.

Alternativ zu Zahnriemen können auch Synchronrundriemen eingesetzt werden, bei denen es sich um Treibriemen mit schraubenartiger Profilierung handelt und die ebenfalls schlupffrei sind. Synchronrundriemen bestehen im Kern aus einem Rundriemen, der wendelförmig von einem Strangelement mit rundem Querschnitt umgeben und fest damit verbunden ist. Die Herstellung von Synchronrundriemen ist zwar aufwändiger als die herkömmlicher Zahnriemen und Synchronrundriemen damit teurer, vorteilhaft ist aufgrund der runden Gestaltung des Riemens keine Schränkung erforderlich, und die dreidimensionale Führung durch das abgewinkelte Armsegment ist bei Verringerung des Platzbedarfs vereinfacht. Die dazu passenden Riemenscheiben weisen an ihrem Umfang eine Rillensegmentierung zum Eingriff mit den der Riemenscheibe zugewandten Windungsabschnitten des wendelförmigen Strangelements und ggf. eine Umfangsnut für den runden Kernriemen auf. Als Alternative zu einem Riemengetriebe ist es ferner möglich, ein Kettengetriebe mit entsprechend an eine Kette angepassten Antriebsscheiben und Rollenelementen als Getriebevorrichtung und/oder Getriebeerweiterung einzusetzen. Außerdem ist es denkbar, dass die Getriebevorrichtung und/oder die Getriebeerweiterung durch Zahnradgetriebe realisiert werden können, wobei Kegelräder zur Kraftübertragung zwischen den abgewinkelten Endabschnitten und dem mittleren Abschnitt eingesetzt werden können.

Die erfindungsgemäße haptische Vorrichtung kann als passives System zur reinen Orientierungs- bzw. Richtungserfassung oder als aktives System für Force-Feedback bzw. Kraftrückkopplung ausgebildet sein, um einem Benutzer eine Kraftrückmeldung zu geben. Die dem Benutzer angezeigten Kräfte und/oder Drehmomente werden mittels kontrollierter Energiezufuhr von einem oder mehreren Aktoren erzeugt, die beispielsweise Capstan-Antriebe oder Direktantriebe auf Basis von DC- oder EC-Motoren sein können.

### BEZUGSZEICHENLISTE

- 1: Handgriff
- 2, 2a: Basisstruktur, Seite
- 3: Erstes Armsegment
- 3a, 3b: Basis-Endabschnitt, Kopplungsendabschnitt
- 4: Zweites Armsegment
- 4a, 4b, 4c: Kopplungsendabschnitt, Arm-Endabschnitt, Zusatzabschnitt
- 5: Drittes Armsegment
- 5a, 5b: Basis-Endabschnitt, Kopplungsendabschnitt
- 6: Viertes Armsegment
- 6a, 6b: Kopplungsendabschnitt, Arm-Endabschnitt
- 7: Erstes Schwenkgelenk
- 8: Zweites Schwenkgelenk
- 9: Drittes Schwenkgelenk
- 10: Haptische Vorrichtung
- 11: Erster Schwenkarm
- 12: Zweiter Schwenkarm
- 13, 13': Getriebevorrichtung
- 130: Zahnriemen
- 131: Erste Riemenscheibe an Basis-Endabschnitt 3a
- 132: Zweite Riemenscheibe an Kopplungsendabschnitt 3b
- 133: Parallele Umlenkrolle
- 134: Orthogonale Umlenkrolle
- 135: Spannrolle
- 136: Zahnung an erster Riemenscheibe 131
- 137: Zweites Zahnrad, verbunden mit zweiter Riemenscheibe 132
- 14: Getriebeerweiterung
- 140: Riemen
- 141: Erste Riemenscheibe an Zusatzabschnitt 4c
- 142: Zweite Riemenscheibe an Arm-Endabschnitt 4b
- 143: Parallele Umlenkrolle
- 144: Orthogonale Umlenkrolle
- 145: Spannrolle
- 146: Zahnung an erster Riemenscheibe 141
- 147: Erstes Zahnrad, verbunden mit erster Riemenscheibe 141
- 15: Handgriffachse
- 16: Taster
- 17: Erstes Kopplungsgelenk
- 18: Zweites Kopplungsgelenk
- 19, 19a: Plattform, Seite

- A: Erste Schwenkachse
- B: Zweite Schwenkachse
- C: Dritte Schwenkachse
- D: Erste Kopplungsachse
- E: Zweite Kopplungsachse
- F: Lagerachse Getriebeerweiterung
- K1, K2: Kreisbahn
- Z: Rotationszentrum

## Patentansprüche

1. Haptische Vorrichtung (10) mit einem Handgriff (1), einer Basisstruktur (2) und einem Gestänge, das den Handgriff (1) mit der Basisstruktur (2) verbindet und drei rotatorische Freiheitsgrade des Handgriffs (1) in Bezug auf die Basisstruktur (2) bereitstellt, wobei das Gestänge zwei Schwenkarme (11, 12) aufweist, wobei ein erster Schwenkarm (11) durch ein erstes Schwenkgelenk (7) mit der Basisstruktur (2) und durch ein zweites Schwenkgelenk (8) mit dem zweiten Schwenkarm (12) verbunden ist, der durch ein drittes Schwenkgelenk (9) mit dem Handgriff (1) verbunden ist, und wobei sich eine erste Schwenkachse (A) des ersten Schwenkgelenks (7), eine zweite Schwenkachse (B) des zweiten Schwenkgelenks (8) und eine dritte Schwenkachse (C) des dritten Schwenkgelenks (9) in einem gemeinsamen Rotationszentrum (Z) schneiden, wobei
- der erste Schwenkarm (11) ein erstes Armsegment (3) mit einem Basis-Endabschnitt (3a) und einem Kopplungsendabschnitt (3b) und ein zweites Armsegment (4) mit einem Kopplungsendabschnitt (4a) und einem Arm-Endabschnitt (4b) aufweist, wobei das erste Schwenkgelenk (7) an dem Basis-Endabschnitt (3a) des ersten Armsegments (3) vorliegt, und wobei
das erste Armsegment (3) und das zweite Armsegment (4) an den Kopplungsendabschnitten (3b, 4a) durch ein erstes Kopplungsgelenk (17) schwenkbar miteinander verbunden und durch eine erste Getriebevorrichtung (13) derart gekoppelt sind, dass der Arm-Endabschnitt (4b) des zweiten Armsegments (4) auf einer ersten Kreisbahn (K1) um eine virtuelle erste Achse bewegt wird, die orthogonal zu der ersten Schwenkachse (A) ist, und
- der zweite Schwenkarm (12) ein drittes Armsegment (5) mit einem Basis-Endabschnitt (5a) und einem Kopplungsendabschnitt (5b) und ein viertes Armsegment (6) mit einem Kopplungsendabschnitt (6a) und einem Arm-Endabschnitt (6b) aufweist, wobei das zweite Schwenkgelenk (8) an dem Arm-Endabschnitt (4b) des zweiten Armsegments (4) und dem Basis-Endabschnitt (5a) des dritten Armsegments (5) und das dritte Schwenkgelenk (9) an dem Arm-Endabschnitt (6b) des vierten Armsegments (6) vorliegen, und wobei
das dritte Armsegment (5) und das vierte Armsegment (6) an den Kopplungsendabschnitten (5b, 6a) durch ein zweites Kopplungsgelenk (18) schwenkbar miteinander verbunden und durch eine zweite Getriebevorrichtung (13') derart gekoppelt sind, dass der Arm-Endabschnitt (6b) des vierten Armsegments (6) auf einer zweiten Kreisbahn (K2) um eine virtuelle zweite Achse bewegt wird, die orthogonal der zweiten Schwenkachse (B) ist, wobei
die Vorrichtung (10) an dem zweiten Armsegment (4) eine Getriebeerweiterung (14) aufweist, die mit der ersten Getriebevorrichtung (13) gekoppelt ist, wobei die Getriebeerweiterung (14) mit einer Plattform (19) gekoppelt ist, die an dem Arm-Endabschnitt (4b) angeordnet und mit dem Basis-Endabschnitt (5a) des dritten Armsegments (5) gekoppelt ist, und wobei die Getriebeerweiterung (14) dazu ausgebildet ist, die Plattform (19) bei Bewegung mit dem Arm-Endabschnitt (4b) entlang der ersten Kreisbahn (K1) in Bezug zu einer Ausrichtung der Basisstruktur (2) konstant auszurichten, sodass die erste virtuelle Achse immer orthogonal zu der zweiten virtuellen Achse ist.

2. Vorrichtung (10) nach Anspruch 1,
**dadurch gekennzeichnet, dass**
ein Kreismittelpunkt der ersten Kreisbahn (K1) des Arm-Endabschnitts (4b) des zweiten Armsegments (4) und
ein Kreismittelpunkt der zweiten Kreisbahn (K2) des Arm-Endabschnitts (6b) des vierten Armsegments (6)
dem gemeinsamen Rotationszentrum (Z) entsprechen.

3. Vorrichtung (10) nach Anspruch 1 oder 2,
**dadurch gekennzeichnet, dass**
jedes Armsegment (3, 4; 5, 6) eine stumpfwinklige V- oder U-Form aufweist, wobei der Basis-Endabschnitt (3a) und der Kopplungsendabschnitt (3b) des ersten Armsegments (3), der Kopplungsendabschnitt (4a) und der Arm-Endabschnitt (4b) des zweiten Armsegments (4), der Basis-Endabschnitt (5a) und der Kopplungsendabschnitt (5b) des dritten Armsegments (5) und der Kopplungsendabschnitt (6a) und der Arm-Endabschnitt (6b) des vierten Armsegments (6) jeweils einen stumpfen Winkel einschließen, wobei
das erste Kopplungsgelenk (17) zwischen dem ersten Armsegment (3) und dem zweiten Armsegment (4) eine erste Kopplungsachse (D) und
das zweite Kopplungsgelenk (18) zwischen dem dritten Armsegment (5) und dem vierten Armsegment (6) eine zweite Kopplungsachse (E) bereitstellen, und sich die erste Kopplungsachse (D) und die zweite Kopplungsachse (E) in dem gemeinsamen Rotationszentrum (Z) schneiden.

4. Vorrichtung (10) nach zumindest einem der Ansprüche 1 bis 3,
**dadurch gekennzeichnet, dass**
die erste Getriebevorrichtung (13) und die zweite Getriebevorrichtung (13') unabhängig voneinander aus einer Gruppe ausgewählt sind, die eine Riemengetriebevorrichtung (13, 13'), eine Kettengetriebevorrichtung, eine Kegelgetriebevorrichtung und Kombinationen davon aufweist.

5. Vorrichtung (10) nach Anspruch 4,
**dadurch gekennzeichnet, dass**
die Riemengetriebevorrichtung (13, 13'), die die erste Getriebevorrichtung (13) bereitstellt, eine erste Riemenscheibe (131) an dem Basis-Endabschnitt (3a) des ersten Armsegments (3), eine zweite Riemenscheibe (132) an dem Kopplungsendabschnitt (3b) des ersten Armsegments (3) und einen Riemen (130) aufweist, der über die erste Riemenscheibe (131) und die zweite Riemenscheibe (132) läuft,
wobei eine Achse der ersten Riemenscheibe (131) an dem Basis-Endabschnitt (3a) des ersten Armsegments (3) der ersten Schwenkachse (A) entspricht, und eine Achse der zweiten Riemenscheibe (132) an dem Kopplungsendabschnitt (3b) des ersten Armsegments (3) der ersten Kopplungsachse (D) entspricht,
und/oder
die Riemengetriebevorrichtung (13, 13'), die die zweite Getriebevorrichtung (13') bereitstellt, eine erste Riemenscheibe an dem Basis-Endabschnitt (5a) des dritten Armsegments (5), eine zweite Riemenscheibe an dem Kopplungsendabschnitt (5b) des dritten Armsegments (5) und einen Riemen aufweist, der über die erste Riemenscheibe und die zweite Riemenscheibe läuft,
wobei eine Achse der ersten Riemenscheibe an dem Basis-Endabschnitt (5a) des dritten Armsegments (5) der zweiten Schwenkachse (B) entspricht, und eine Achse der zweiten Riemenscheibe an dem Kopplungsendabschnitt (5b) des dritten Armsegments (5) der zweiten Kopplungsachse (E) entspricht.

6. Vorrichtung (10) nach Anspruch 5,
**dadurch gekennzeichnet, dass**
der Riemen (130) der Riemengetriebevorrichtung (13, 13') ein Profil aufweist und aus einer Gruppe ausgewählt ist, die zumindest einen Zahnriemen (130) und einen Synchronrundriemen, der ein mit einem Wendelelement verbundener Rundriemen ist, umfasst, und
die erste Riemenscheibe (131) und die zweite Riemenscheibe (132) eine Profilierung aufweisen, die mit dem Profil des Riemens (130) korrespondiert und die aus einer Gruppe ausgewählt ist, die zumindest eine Zahnung (136) zum Eingriff mit dem Zahnriemen (130) und eine Rillensegmentierung zum Eingriff dem Synchronrundriemen umfasst.

7. Vorrichtung (10) nach Anspruch 5 oder 6,
**dadurch gekennzeichnet, dass**
die Riemengetriebevorrichtung (13, 13') zumindest ein Rollenelement aufweist, das zur Führung des Riemens (130) zwischen der ersten Riemenscheibe (131) und der zweiten Riemenscheibe (132) ausgebildet und aus einer Gruppe ausgewählt ist, die zumindest eine Umlenkrolle (133, 134) und eine Spannrolle (135) umfasst.

8. Vorrichtung (10) nach zumindest einem der Ansprüche 4 bis 7,
**dadurch gekennzeichnet, dass**
die Getriebeerweiterung (14) aus einer Gruppe ausgewählt ist, die eine Riemengetriebevorrichtung, eine Kettengetriebevorrichtung, eine Zahnradgetriebevorrichtung, eine kombinierte Riemen-Zahnradgetriebevorrichtung (14) und eine kombinierte Ketten-Zahnradgetriebevorrichtung aufweist.

9. Vorrichtung (10) nach Anspruch 8,
**dadurch gekennzeichnet, dass**
die kombinierte Riemen-Zahnradgetriebevorrichtung (14), die die Getriebeerweiterung (14) bereitstellt,
- eine erste Riemenscheibe (141), die an einem Zusatzabschnitt (4c) des zweiten Armsegments (4) auf einer von dem Arm-Endabschnitt (4b) abgewandten Seite des Kopplungsendabschnitts (4a) angeordnet ist, eine zweite Riemenscheibe (142) an dem Arm-Endabschnitt (4b) des zweiten Armsegments (4) und einen Riemen (140) aufweist, der über die erste Riemenscheibe (141) und die zweite Riemenscheibe (142) läuft,
wobei eine Achse (F) der ersten Riemenscheibe (141) an dem Zusatzabschnitt (4c) des zweiten Armsegments (4) parallel zu der ersten Kopplungsachse (D) ist, und eine Achse der zweiten Riemenscheibe (142) an dem Arm-Endabschnitt (4b) des zweiten Armsegments (4) der zweiten Schwenkachse (B) entspricht,
und
- ein erstes Zahnrad (147) aufweist, das koaxial mit der ersten Riemenscheibe (141) drehfest verbunden ist und mit einem zweiten Zahnrad (137) in Eingriff steht, das koaxial mit der zweiten Riemenscheibe (132) der ersten Getriebevorrichtung (13) drehfest verbunden ist.

10. Vorrichtung (10) nach Anspruch 9,
**dadurch gekennzeichnet, dass**
der Riemen (140) der kombinierten Riemen-Zahnradgetriebevorrichtung (14), die die Getriebeerweiterung (14) bereitstellt, ein Profil aufweist und aus einer Gruppe ausgewählt ist, die zumindest einen Zahnriemen (140) und einen Synchronrundriemen, der ein mit einem Wendelelement verbundener Rundriemen ist, umfasst, und die erste Riemenscheibe (141) und die zweite Riemenscheibe (142) eine Profilierung aufweisen, die mit dem Profil des Riemens (140) korrespondiert und die aus einer Gruppe ausgewählt ist, die zumindest eine Zahnung (146) zum Eingriff mit dem Zahnriemen (140) und eine Rillensegmentierung zum Eingriff dem Synchronrundriemen umfasst.

11. Vorrichtung (10) nach Anspruch 9 oder 10,
**dadurch gekennzeichnet, dass**
die kombinierte Riemen-Zahnradgetriebevorrichtung (14) zumindest ein Rollenelement aufweist, das zur Führung des Riemens (140) zwischen der ersten Riemenscheibe (141) und der zweiten Riemenscheibe (142) ausgebildet und aus einer Gruppe ausgewählt ist, die zumindest eine Umlenkrolle (143, 144) und eine Spannrolle (145) umfasst.

## Claims

1. A haptic device (10) with a handle (1), a base structure (2) and a linkage which connects the handle (1) to the base structure (2) and provides three rotational degrees of freedom of the handle (1) in relation to the base structure (2), wherein the linkage has two pivot arms (11, 12), wherein a first pivot arm (11) is connected to the base structure (2) by a first pivot joint (7) and to the second pivot arm (12) by a second pivot joint (8), which second pivot arm is connected to the handle (1) by a third pivot joint (9), and wherein a first pivot axis (A) of the first pivot joint (7), a second pivot axis (B) of the second pivot joint (8) and a third pivot axis (C) of the third pivot joint (9) intersect in a common centre of rotation (Z),
wherein
- the first pivot arm (11) has a first arm segment (3) with a base end section (3a) and a coupling end section (3b) and a second arm segment (4) with a coupling end section (4a) and an arm end section (4b), wherein the first pivot joint (7) is present at the base end section (3a) of the first arm segment (3), and wherein
the first arm segment (3) and the second arm segment (4) are pivotably connected to each other at the coupling end sections (3b, 4a) by a first coupling joint (17) and are coupled by a first transmission mechanism (13) in such manner that the arm end section (4b) of the second arm segment (4) is moved on a first circular path (K1) about a virtual first axis which is orthogonal to the first pivot axis (A), and
- the second pivot arm (12) has a third arm segment (5) with a base end section (5a) and a coupling end section (5b) and a fourth arm segment (6) with a coupling end section (6a) and an arm end section (6b), wherein the second pivot joint (8) is present at the arm end section (4b) of the second arm segment (4) and the base end section (5a) of the third arm segment (5), and the third pivot joint (9) is present at the arm end section (6b) of the fourth arm segment (6), and wherein
the third arm segment (5) and the fourth arm segment (6) are pivotably connected to each other at the coupling end sections (5b, 6a) by a second coupling joint (18) and are coupled by a second transmission mechanism (13') in such manner that the arm end section (6b) of the fourth arm segment (6) is moved on a second circular path (K2) about a virtual second axis which is orthogonal to the second pivot axis (B), wherein
the device (10) has, on the second arm segment (4), a transmission extension (14) which is coupled to the first transmission mechanism (13), wherein the transmission extension (14) is coupled to a platform (19) which is arranged on the arm end section (4b) and is coupled to the base end section (5a) of the third arm segment (5) and wherein the transmission extension (14) is designed to constantly align the platform (19) during movement with the arm end section (4b) along the first circular path (K1) in relation to an alignment of the base structure (2) such that the first virtual axis is always orthogonal to the second virtual axis.

2. The device (10) according to claim 1,
**characterised in that**
a circle centre of the first circular path (K1) of the arm end section (4b) of the second arm segment (4) and
a circle centre of the second circular path (K2) of the arm end section (6b) of the fourth arm segment (6)
correspond to the common centre of rotation (Z).

3. The device (10) according to claim 1 or 2,
**characterised in that**
each arm segment (3, 4; 5, 6) has an obtuse-angled V- or U-shape, wherein the base end section (3a) and the coupling end section (3b) of the first arm segment (3), the coupling end section (4a) and the arm end section (4b) of the second arm segment (4) the base end section (5a) and the coupling end section (5b) of the third arm segment (5) and the coupling end section (6a) and the arm end section (6b) of the fourth arm segment (6) each form an obtuse angle, wherein
the first coupling joint (17) has a first coupling axis (D) between the first arm segment (3) and the second arm segment (4) and
the second coupling joint (18) provides a second coupling axis (E) between the third arm segment (5) and the fourth arm segment (6), and the first coupling axis (D) and the second coupling axis (E) intersect in the common centre of rotation (Z).

4. The device (10) according to at least one of claims 1 to 4,
**characterised in that**
the first transmission mechanism (13) and the second transmission mechanism (13') are selected independently of each other from a group which includes a belt transmission mechanism (13, 13'), a chain transmission mechanism, a bevel gear transmission mechanism and combinations thereof.

5. The device (10) according to claim 4,
**characterised in that**
the belt transmission mechanism (13, 13'), which provides the first transmission mechanism (13), has a first belt pulley (131) at the base end section (3a) of the first arm segment (3), a second belt pulley (132) at the coupling end section (3b) of the first arm segment (3), and a belt (130), which runs via the first belt pulley (131) and the second belt pulley (132),
wherein an axis of the first belt pulley (131) at the base end section (3a) of the first arm segment (3) corresponds to the first pivot axis (A), and an axis of the second belt pulley (132) at the coupling end section (3b) of the first arm segment (3) corresponds to the first coupling axis (D),
and/or
the belt transmission mechanism (13, 13'), which provides the second transmission mechanism (13'), has a first belt pulley at the base end section (5a) of the third arm segment (5), a second belt pulley at the coupling end section (5b) of the third arm segment (5), and a belt, which runs via the first belt pulley and the second belt pulley,
wherein an axis of the first belt pulley at the base end section (5a) of the third arm segment (5) corresponds to the second pivot axis (B), and an axis of the second belt pulley at the coupling end section (5b) of the third arm segment (5) corresponds to the second coupling axis (E).

6. The device (10) according to claim 5,
**characterised in that**
the belt (130) of the belt transmission mechanism (13, 13') has a profile and is selected from a group which comprises at least one toothed belt (130) and one synchronous round belt, which is a round belt connected to a helical element, and the first belt pulley (131) and the second belt pulley (132) have a profiling which corresponds to the profile of the belt (130) and which is selected from a group which comprises at least one toothing (136) for engagement with the toothed belt (130) and one groove segmentation for engagement with the synchronous round belt.

7. The device (10) according to claim 5 or 6,
**characterised in that**
the belt transmission mechanism (13, 13') has at least one roller element which is designed to guide the belt (130) between the first belt pulley (131) and the second belt pulley (132) and is selected from a group which comprises at least one deflection roller (133, 134) and one tension roller (135).

8. The device (10) according to at least one of claims 4 to 7,
**characterised in that**
the transmission extension (14) is selected from a group which includes a belt transmission mechanism, a chain transmission mechanism, a gear transmission mechanism, a combined belt-gear transmission mechanism (14) and a combined chain-gear transmission mechanism.

9. The device (10) according to claim 8,
**characterised in that**
the combined belt-gear transmission mechanism (14), which provides the transmission extension (14),
- has a first belt pulley (141), which is arranged on an additional section (4c) of the second arm segment (4) on a side of the coupling end section (4a) facing away from the arm end section (4b), has a second belt pulley (142) on the arm end section (4b) of the second arm segment (4), and has a belt (140), which runs via the first belt pulley (141) and the second belt pulley (142),
wherein an axis (F) of the first belt pulley (141) at the additional section (4c) of the second arm segment (4) is parallel to the first coupling axis (D), and an axis of the second belt pulley (142) at the arm end section (4b) of the second arm segment (4) corresponds to the second pivot axis (B),
and
- has a first gear (147) which is coaxially connected to the first belt pulley (141) in a rotationally-fixed manner and engages with a second gear (137) which is coaxially connected to the second belt pulley (132) of the first transmission mechanism (13) in a rotationally-fixed manner.

10. The device (10) according to claim 9,
**characterised in that**
the belt (140) of the combined belt-gear transmission mechanism (14), which provides the transmission extension (14), has a profile and is selected from a group which comprises at least one toothed belt (140) and one synchronous round belt, which is a round belt connected to a helical element, and the first belt pulley (141) and the second belt pulley (142) have a profiling which corresponds to the profile of the belt (140) and which is selected from a group which comprises at least one toothing (146) for engagement with the toothed belt (140) and one groove segmentation for engagement with the synchronous round belt.

11. The device (10) according to claim 9 or 10,
**characterised in that**
the combined belt-gear transmission mechanism (14) has at least one roller element which is designed to guide the belt (140) between the first belt pulley (141) and the second belt pulley (142) and is selected from a group which comprises at least one deflection roller (143, 144) and one tension roller (145).

## Revendications

1. Dispositif haptique (10) comprenant une poignée (1), une structure de base (2) et une tringlerie reliant la poignée (1) à la structure de base (2) et fournissant trois degrés de liberté pour la rotation de la poignée (1) par rapport à la structure de base (2), dans laquelle la tringlerie comprend deux bras pivotants (11, 12), un premier bras pivotant (11) étant relié à la structure de base (2) par une première articulation pivotante (7) et au deuxième bras pivotant (12) par une deuxième articulation pivotante (8), qui est relié à la poignée (1) par une troisième articulation pivotante (9), et dans lequel un premier axe de pivotement (A) de la première articulation pivotante (7), un deuxième axe de pivotement (B) de la deuxième articulation pivotante (8) et un troisième axe de pivotement (C) de la troisième articulation pivotante (9) se coupent en un centre de rotation commun (Z),
dans laquelle
- le premier bras pivotant (11) comprend un premier segment de bras (3) ayant une partie d'extrémité de base (3a) et une partie d'extrémité de couplage (3b), et un second segment de bras (4) ayant une partie d'extrémité de couplage (4a) et une partie d'extrémité de bras (4b), dans lequel la première articulation pivotante (7) se trouve sur la partie d'extrémité de base (3a) du premier segment de bras (3), et dans lequel
le premier segment de bras (3) et le deuxième segment de bras (4) sont reliés de manière pivotante l'un à l'autre au niveau des parties de l'extrémité du couplage (3b, 4a) par une première articulation de couplage (17) et sont couplés par un premier dispositif de transmission (13) de telle sorte que la partie d'extrémité de bras (4b) du deuxième segment de bras (4) est déplacée sur une première trajectoire circulaire (K1) autour d'un premier axe virtuel qui est orthogonal au premier axe de pivotement (A), et
- le deuxième bras pivotant (12) comprend un troisième segment de bras (5) ayant une partie d'extrémité de base (5a) et une partie d'extrémité de couplage (5b) et un quatrième segment de bras (6) ayant une partie d'extrémité de couplage (6a) et une partie d'extrémité de bras (6b), dans lequel la deuxième articulation pivotante (8) se trouve sur la partie d'extrémité de bras (4b) du deuxième segment de bras (4) et la partie d'extrémité de base (5a) du troisième segment de bras (5) et la troisième articulation pivotante (9) se trouve sur la partie d'extrémité de bras (6b) du quatrième segment de bras (6), et dans lequel
le troisième segment de bras (5) et le quatrième segment de bras (6) sont reliés de manière pivotante l'un à l'autre au niveau des parties d'extrémité de couplage (5b, 6a) par une deuxième articulation de couplage (18) et sont couplés par un deuxième dispositif de transmission (13') de telle sorte que la partie d'extrémité de bras (6b) du quatrième segment de bras (6) est déplacée sur une deuxième trajectoire circulaire (K2) autour d'un deuxième axe virtuel qui est orthogonal au deuxième axe de pivotement (B), dans lequel
le dispositif (10) comprend, sur le deuxième segment de bras (4), une extension de transmission (14) couplée au premier dispositif de transmission (13), l'extension de transmission (14) étant couplée à une plateforme (19) disposée sur la partie d'extrémité de bras (4b) et couplée à la partie d'extrémité de base (5a) du troisième segment de bras (5), et dans lequel l'extension d'engrenage (14) est adaptée pour orienter la plateforme (19) de manière constante par rapport à une orientation de la structure de base (2) lorsqu'elle se déplace avec la partie d'extrémité de bras (4b) le long de la première trajectoire circulaire (K1), de sorte que le premier axe virtuel est toujours orthogonal par rapport au deuxième axe virtuel.

2. Dispositif (10) selon la revendication 1,
**caractérisé en ce que**
un centre de cercle de la première trajectoire circulaire (K1) de la partie d'extrémité de bras (4b) du deuxième segment de bras (4), et
un centre de cercle de la deuxième trajectoire circulaire (K2) de la partie d'extrémité de bras (6b) du quatrième segment de bras (6) correspondent au centre de rotation commun (Z).

3. Dispositif (10) selon la revendication 1 ou 2,
**caractérisé en ce que**
chaque segment de bras (3, 4 ; 5, 6) a une forme en V ou en U à angle obtus, la partie d'extrémité de base (3a) et la partie d'extrémité de couplage (3b) du premier segment de bras (3), la partie d'extrémité de couplage (4a) et la partie d'extrémité de bras (4b) du deuxième segment de bras (4), la partie d'extrémité de base (5a) et la partie d'extrémité de couplage (5b) du troisième segment de bras (5) et la partie d'extrémité de couplage (6a) et la partie d'extrémité de bras (6b) du quatrième segment de bras (6) forment chacune un angle obtus, dans lequel
la première articulation de couplage (17) entre le premier segment de bras (3) et le deuxième segment de bras (4) a un premier axe de couplage (D) et
la deuxième articulation de couplage (18) fournit un deuxième axe de couplage (E) entre le troisième segment de bras (5) et le quatrième segment de bras (6), et le premier axe de couplage (D) et le deuxième axe de couplage (E) se coupent au niveau du centre de rotation commun (Z).

4. Dispositif (10) selon au moins l'une des revendications 1 à 4,
**caractérisé en ce que**
le premier dispositif d'engrenage (13) et le deuxième dispositif d'engrenage (13') sont choisis indépendamment l'un de l'autre dans un groupe comprenant un dispositif d'engrenage à courroie (13, 13'), un dispositif d'engrenage à chaîne, un dispositif d'engrenage conique, et des combinaisons de ceux-ci.

5. Dispositif (10) selon la revendication 4,
**caractérisé en ce que**
le dispositif de transmission à courroie (13, 13') fournissant le premier dispositif de transmission (13) comprend une première poulie (131) au niveau de la partie d'extrémité de base (3a) du premier segment de bras (3), une deuxième poulie (132) au niveau de la partie d'extrémité de couplage (3b) du premier segment de bras (3), et une courroie (130) passant sur la première poulie (131) et la deuxième poulie (132),
dans lequel un axe de la première poulie (131) au niveau de la partie d'extrémité de base (3a) du premier segment de bras (3) correspond au premier axe de pivotement (A), et un axe de la deuxième poulie (132) au niveau de la partie d'extrémité de couplage (3b) du premier segment de bras (3) correspond au premier axe de couplage (D),
et/ou
le dispositif de transmission à courroie (13, 13') fournissant le deuxième dispositif de transmission (13') comprend une première poulie au niveau de la partie d'extrémité de base (5a) du troisième segment de bras (5), une deuxième poulie au niveau de la partie d'extrémité de couplage (5b) du troisième segment de bras (5), et une courroie passant sur la première poulie et la deuxième poulie,
dans lequel un axe de la première poulie au niveau de la partie d'extrémité de base (5a) du troisième segment de bras (5) correspond au deuxième axe de pivotement (B), et un axe de la deuxième poulie au niveau de la partie d'extrémité de couplage (5b) du troisième segment de bras (5) correspond au deuxième axe de couplage (E).

6. Dispositif (10) selon la revendication 5,
**caractérisé en ce que**
la courroie (130) du dispositif de transmission à courroie (13, 13') a un profil et est choisie dans un groupe comprenant au moins une courroie crantée (130) et une courroie ronde synchrone qui est une courroie ronde reliée à un élément de retournement, et
la première poulie (131) et la deuxième poulie (132) ont un profil qui correspond au profil de la courroie (130) et qui est choisi dans un groupe comprenant au moins une denture (136) destinée à s'engager avec la courroie crantée (130) et une segmentation à rainures destinée à s'engager avec la courroie ronde synchrone.

7. Dispositif (10) selon la revendication 5 ou 6,
**caractérisé en ce que**
le dispositif de transmission par courroie (13, 13') comprend au moins un élément de rouleau configuré pour guider la courroie (130) entre la première poulie (131) et la deuxième poulie (132) et sélectionné dans un groupe comprenant au moins un rouleau de renvoi (133, 134) et un rouleau de tension (135).

8. Dispositif (10) selon au moins l'une des revendications 4 à 7,
**caractérisé en ce que**
l'extension de transmission (14) est choisie dans un groupe comprenant un dispositif de transmission à courroie, un dispositif de transmission à chaîne, un dispositif de transmission à engrenage, un dispositif d'engrenage combiné à courroie et à engrenage (14) et un dispositif de transmission combiné à chaîne et à engrenage.

9. Dispositif (10) selon la revendication 8,
**caractérisé en ce que**
dispositif d'engrenage combiné à courroie et à engrenage (14) fournissant l'extension de transmission (14),
- une première poulie (141) disposée sur une partie auxiliaire (4c) du deuxième segment de bras (4) sur un côté de la partie d'extrémité de couplage (4a) éloigné de la partie d'extrémité de bras (4b), une seconde poulie (142) sur la partie d'extrémité de bras (4b) du deuxième segment de bras (4), et une courroie (140) passant sur la première poulie (141) et la seconde poulie (142),
dans lequel un axe (F) de la première poulie (141) sur la partie auxiliaire (4c) du deuxième segment de bras (4) est parallèle au premier axe de couplage (D), et un axe de la deuxième poulie (142) sur la partie d'extrémité de bras (4b) du deuxième segment de bras (4) correspond au deuxième axe de pivotement (B), et
- une première roue dentée (147) reliée coaxialement à la première poulie (141) de manière à ce que toute rotation soit impossible et engrenant avec une deuxième roue dentée (137) reliée coaxialement à la seconde poulie (132) du premier dispositif de transmission (13) de manière à ce que toute rotation soit impossible.

10. Dispositif (10) selon la revendication 9,
**caractérisé en ce que**
la courroie (140) du dispositif d'engrenage combiné à courroie et à engrenage (14) fournissant l'extension d'engrenage (14) a un profil et est sélectionnée dans un groupe comprenant au moins une courroie dentée (140) et une courroie ronde synchrone qui est une courroie ronde reliée à un élément de retournement, et la première poulie (141) et la deuxième poulie (142) ont un profil correspondant au profil de la courroie (140) et sont sélectionnées dans un groupe comprenant au moins une denture (146) destinée à s'engrener avec la courroie dentée (140) et une segmentation à rainures destinée à s'engrener avec la courroie ronde synchrone.

11. Dispositif (10) selon la revendication 9 ou 10,
**caractérisé en ce que**
dispositif d'engrenage combiné à courroie et à engrenage (14) comprend au moins un élément de poulie configuré pour guider la courroie (140) entre la première poulie (141) et la deuxième poulie (142) et sélectionné dans un groupe comprenant au moins une poulie de renvoi (143, 144) et une poulie de tension (145).
